(19) Europäisches Patentamt
European Patent Office
Office européen des brevets

(11) EP 4 781 931 A1

(12) **EUROPEAN PATENT APPLICATION**
published in accordance with Art. 153(4) EPC

(43) Date of publication:
29.07.2026 Bulletin 2026/31

(21) Application number: 24868166.0

(22) Date of filing: 10.09.2024

(51) International Patent Classification (IPC):
A61B 18/12 (2006.01)    A61B 18/14 (2006.01)

(52) Cooperative Patent Classification (CPC):
A61B 18/12; A61B 18/14

(86) International application number:
PCT/JP2024/032326

(87) International publication number:
WO 2025/063088 (27.03.2025 Gazette 2025/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.09.2023 JP 2023155455

(71) Applicant: Terumo Kabushiki Kaisha
Tokyo 151-0072 (JP)

(72) Inventor: TAKAHASHI Yusuke
Ashigarakami-gun, Kanagawa 259-0151 (JP)

(74) Representative: Prüfer & Partner mbB
Patentanwälte · Rechtsanwälte
Sohnckestraße 12
81479 München (DE)

(54) **MEDICAL DEVICE AND METHOD FOR FORMING COMMUNICATION HOLE**

(57) To provide a medical device that reduces the likelihood of the occurrence of recoil or remodeling by appropriately setting a region of a biological tissue to be cauterized by an electrode unit. The present invention provides a medical device 10 for forming a communication hole Hh in biological tissue, the medical device 10 including: an expansion body 21 including wire materials 50 and expandable and contractible in the radial direction; and electrode units 22 disposed on the expansion body 21, wherein the number N of the electrode units 22, the length L (mm) of each of the electrode units 22 along the direction of extension of the wire materials 50, and the width W (mm) of each of the electrode units 22 orthogonal to the length L (mm) satisfy (Mathematical Expression 1) and (Mathematical Expression 2).

[Mathematical Expression 1]

$$L \leq 2.0$$

[Mathematical Expression 2]

$$\frac{(W + 1) \times N}{8\pi} \geq 0.40$$

FIG. 7

## Description

Technical Field

The present invention relates to a medical device that applies energy to biological tissue and a method for forming a communication hole in an atrial septum using the medical device.

Background Art

As a medical device, a device that performs a treatment by ablation to cauterize biological tissue by a high-frequency current from an electrode unit disposed on an expansion body which is expanded and contracted in a living body is known. As one of treatments by ablation, a shunt treatment on the atrial septum is known. The shunt treatment can alleviate heart failure symptoms of a patient with heart failure by forming a shunt (communication hole) serving as an escape route for an increased atrial pressure in the fossa ovalis of the atrial septum of the patient. In the shunt treatment, the atrial septum is accessed using an intravenous approaching method, and a shunt with a desired size is formed.

The expansion body has a recess that is recessed radially inward during expansion of the expansion body to define a reception space capable of receiving the biological tissue, and can hold the biological tissue from both sides in the thickness direction. The electrode unit is disposed in the recess. Such a medical device is disclosed in, for example, Patent Literature 1.

Citation List

Patent Literature

Patent Literature 1: WO 2020-094094

Summary of Invention

Technical Problem

In a medical device that applies energy to a biological tissue, when a region of the biological tissue to be cauterized by the electrode unit is too narrow, recoil may occur in which the formed shunt contracts in the short term. On the other hand, when the region of the biological tissue to be cauterized by the electrode unit is too wide, a strong healing reaction of the living body is generated, so that remodeling may occur in which the shunt is blocked in the long term. In view of this, it is necessary to appropriately set the region of the biological tissue to be cauterized by the electrode unit.

The present invention has been made to solve the above-described problems, and an object of the present invention is to provide a medical device that reduces the likelihood of the occurrence of recoil or remodeling by appropriately setting a region of a biological tissue to be cauterized by an electrode unit, and a method for forming a communication hole using the medical device.

Solution to Problem

In order to achieve the above-mentioned object, (1) a medical device according to the present invention is for forming a communication hole in a biological tissue, the medical device including: an expansion body that is expandable and contractible in a radial direction; and a plurality of electrode units disposed on the expansion body, wherein the expansion body has a recess that is recessed radially inward upon expansion of the expansion body and that defines a reception space capable of receiving the biological tissue, the recess includes a bottom that is an innermost part in the radial direction of the expansion body, a proximal-side upright portion extending radially outward from a proximal end of the bottom, and a distal-side upright portion extending radially outward from a distal end of the bottom, the plurality of electrode units extends along the proximal-side upright portion or the distal-side upright portion and is spaced apart from each other in a circumferential direction of the expansion body, each of the plurality of electrode units includes a bottom-side end located on a side of the bottom of the recess and an outer end located on a side opposite to the bottom-side end in the radial direction of the expansion body, and has a length L extending along the distal-side upright portion or the proximal-side upright portion from the bottom-side end to the outer end, and a width W (mm) orthogonal to the length L (mm), and a number N of the electrode units, the length L (mm) of each of the plurality of electrode units, and an average width Wa (mm) that is an average of the widths W (mm) of the plurality of electrode units satisfy (Mathematical Expression 1) and (Mathematical Expression 2).

[Mathematical Expression 1]

$$L \leq 2.0$$

[Mathematical Expression 2]

$$\frac{(W + 1) \times N}{8\pi} \geq 0.40$$

In order to achieve the above-mentioned object, (9) a method for forming a communication hole according to the present invention is a method for forming a communication hole that allows communication between a right atrium and a left atrium in an atrial septum using an expansion body that is expandable and contractible in a radial direction, the method comprising: preparing the expansion body including a recess that is recessed radially inward upon expansion of the expansion body and that defines a reception space capable of receiving a biological tissue; inserting the expansion body into a first through hole formed in the atrial septum to place a tissue surrounding the first through hole in the reception space defined by the recess; expanding the recess of the expansion body in the radial direction to expand the first through hole to a second through hole larger than the communication hole; cauterizing the tissue surrounding the second through hole with a plurality of electrode units disposed in the recess of the expansion body in such a manner that a cauterization region of the tissue surrounding the communication hole has a length of 3.0 mm or less extending in the radial direction from an edge of the communication hole and a ratio of 40% or more to a circumferential length of the edge of the communication hole in a circumferential direction; and forming the communication hole by contracting and removing the expansion body from the second communication hole after the cauterization of the tissue surrounding the second through hole.

Advantageous Effects of Invention

In the (1) medical device configured as described above, the electrode units can cauterize an appropriate region around the communication hole, whereby it is possible to reduce the possibility of the occurrence of recoil in which the formed shunt contracts in the short term or remodeling in which the shunt is blocked in the long term due to strong healing reaction of a living body.

(2) In the medical device according to (1), the number N of the electrode units and the average width Wa (mm) of the plurality of electrode units may satisfy (Mathematical Expression 3). With this configuration, the medical device cauterizes a wider region around the communication hole, whereby it is possible to more reliably prevent the formed shunt from contracting in the short term.

[Mathematical Expression 3]

$$\frac{(W + 1) \times N}{8\pi} \geq 0.60$$

(3) In the medical device according to (1) or (2), the number N of the electrode units may be ten or more. With this configuration, the medical device can satisfy the condition represented by the above expression even if the width of each electrode unit is reduced, so that the flexibility of the expansion body can be increased.

(4) In the medical device according to any one of (1) to (3), the recess may be deformed so as to crush and hold the biological tissue in a state in which the biological tissue is received in the reception space. With this configuration, the medical device can cauterize the biological tissue in an appropriate region when the biological tissue is held by the recess so as to be crushed and is cauterized by the electrode units.

(5) In the medical device according to any one of (1) to (4), the length L (mm) of each of the plurality of electrode units may satisfy (Mathematical Expression 4). With this configuration, the medical device can reduce the possibility of occurrence of remodeling while sufficiently ensuring the area to be cauterized by the electrode units.

[Mathematical Expression 4]

$$0.7 \leq L \leq 2.0$$

(6) In the medical device according to any one of (1) to (5), each of the plurality of electrode units may be disposed in such a manner that the bottom-side end is in contact with the radially outer surface of the bottom of the recess, and the length L (mm) of each of the plurality of electrode units may satisfy (Mathematical Expression 5). With this configuration, the region to be cauterized by the electrode units can be reliably kept within the region of the fossa ovalis.

[Mathematical Expression 5]

$$L \leq 1.5$$

(7) In the medical device according to any one of (1) to (6), the plurality of electrode units may be disposed at substantially equal intervals along the circumferential direction of the expansion body. With this configuration, the cauterization can be performed at equal intervals along the circumferential direction by the electrode units, whereby recoil that is short-term contraction can be further reduced.

(8) In the medical device according to any one of (1) to (7), in a state in which the expansion body is maximally expanded, a distance from a central axis of the expansion body in the radial direction of the expansion body to the outer end of each of the plurality of electrode units may be within a range of 9 mm to 11 mm. With this configuration, it is possible to form a communication hole having a diameter of about 8 mm immediately after cauterization while keeping the region to be cauterized by the electrode units approximately within the region of the fossa ovalis.

With the (9) method for forming the communication hole configured as described above, an appropriate region suitable for the diameter of the communication hole slightly contracted from the second through hole after the removal of the expansion body can be cauterized by the electrode units. Thus, it is possible to reduce the possibility of the occurrence of recoil in which the formed shunt contracts in the short term or remodeling in which the shunt is blocked in the long term due to strong healing reaction of the living body.

Brief Description of Drawings

[Fig. 1] Fig. 1 is a front view illustrating an overall configuration of a medical device according to an embodiment.
[Fig. 2] Fig. 2 is an enlarged perspective view illustrating the vicinity of an expansion body.
[Fig. 3] Fig. 3 is an enlarged front view of the vicinity of the expansion body.
[Fig. 4] Fig. 4 is a flowchart illustrating a procedure for forming a communication hole.
[Fig. 5] Fig. 5 is a cross-sectional view of a first through hole.
[Fig. 6] Fig. 6 is an explanatory diagram schematically illustrating a state where the expansion body is placed in an atrial septum, and illustrates a front view of the medical device and a cross-sectional view of a biological tissue.
[Fig. 7] Fig. 7 is an enlarged front view illustrating a state in which the expansion body grips a biological tissue.
[Fig. 8] Fig. 8 is a cross-sectional view of a communication hole that has been formed.
[Fig. 9] Fig. 9 is a diagram illustrating a positional relationship between a second through hole in a biological tissue and electrode units in a state where the expansion body grips the biological tissue.
[Fig. 10] Fig. 10 is a graph showing a relationship between a cauterization ratio, which is the ratio of the total of widths of regions to be cauterized by the electrode unit to the circumferential length of the communication hole, and an area shrinkage rate of the communication hole after a certain period of time has elapsed from the cauterization.
[Fig. 11] Fig. 11 is an enlarged perspective view of the vicinity of an expansion body having six electrode units.
[Fig. 12] Fig. 12 is a diagram illustrating a positional relationship between a second through hole in a biological tissue and the electrode units in a state where the expansion body in Fig. 9 grips the biological tissue.
[Fig. 13] Fig. 13 is an enlarged perspective view of the vicinity of an expansion body having four electrode units.
[Fig. 14] Fig. 14 is a diagram illustrating a positional relationship between a second through hole in a biological tissue and the electrode units in a state where the expansion body in Fig. 11 grips the biological tissue.

Description of Embodiments

Hereinafter, an embodiment of the present invention will be described with reference to the drawings. Note that dimensional ratios in the drawings may be exaggerated and different from actual ratios for convenience of description. In addition, in the present specification, a side of a medical device 10 that is to be inserted into a biological lumen will be referred to as a "distal end" or a "distal side", and a side near the operator's hand operating the medical device 10 will be referred to as a "proximal end" or a "proximal side".

The medical device according to the embodiment described below is configured to expand a first through hole Hh formed in an atrial septum HA of the heart H of a patient to form a second through hole Hh2, and to further perform maintenance procedure to maintain the expanded second through hole Hh2 at the increased size to obtain a communication hole Hh.

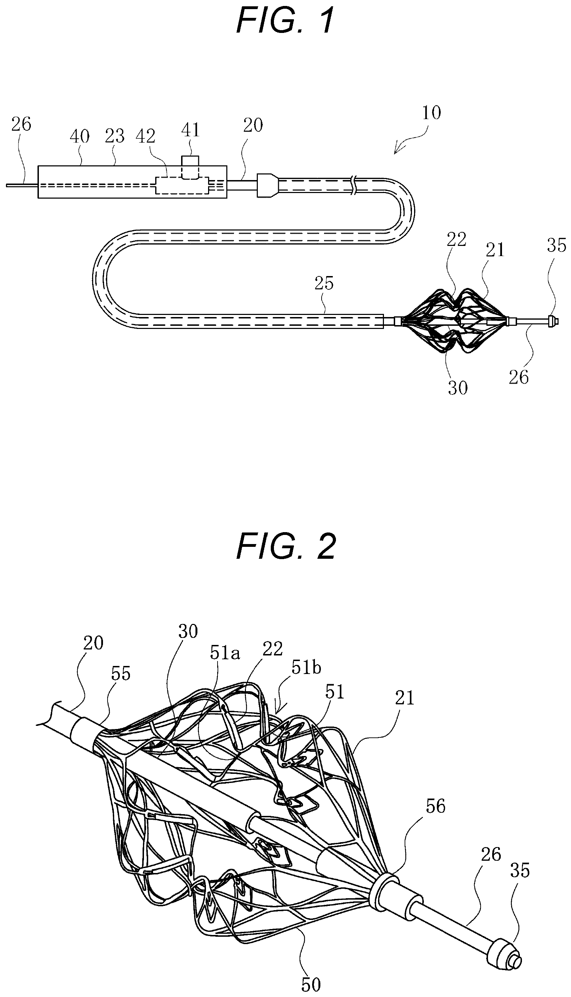

As illustrated in Fig. 1, the medical device 10 according to the present embodiment includes an elongated shaft portion 20, an expansion body 21 disposed on a distal part of the shaft portion 20, and a hand operation unit 23 disposed on a proximal part of the shaft portion 20. The expansion body 21 has an electrode unit 22 which is an energy transfer element for performing the above-described maintenance procedure.

The shaft portion 20 has a distal shaft portion 30 extending to the inside of the expansion body 21 at the distal part. The distal shaft portion 30 extends along a central axis of the expansion body 21 from the vicinity of the proximal end of the expansion body 21 to the middle of the expansion body 21, specifically, to the vicinity of a recess 51 of the expansion body 21 to be described later.

The shaft portion 20 includes a storage sheath 25 disposed on an outermost peripheral portion. The expansion body 21 is movable forward and rearward in an axial direction with respect to the storage sheath 25. The storage sheath 25 can store the expansion body 21 therein in a state of moving to the distal side of the shaft portion 20. The expansion body 21 can be exposed from the storage sheath 25 by moving the storage sheath 25 that has stored the expansion body 21 to the proximal side.

A pulling shaft 26 is disposed in the shaft portion 20 so as to be slidable with respect to the shaft portion 20. The pulling shaft 26 is disposed from a position proximal of the hand operation unit 23 to a position distal of the expansion body 21. The pulling shaft 26 protrudes from the distal part of the shaft portion 20, specifically, from the distal shaft portion 30, passes through the inside of the expansion body 21, and protrudes from the distal end of the expansion body 21. A distal part of the pulling shaft 26 is fixed to a distal end member 35.

The distal end member 35 to which the distal part of the pulling shaft 26 is fixed need not be fixed to the expansion body 21. As a result, when the pulling shaft 26 slides in a proximal direction with respect to the shaft portion 20, the distal end member 35 can apply a compressive force to the expansion body 21 along the axis of the shaft portion 20. In addition, when the expansion body 21 is stored in the storage sheath 25, the distal end member 35 is moved away from the expansion body 21 to the distal side, by which the expansion body 21 can be rather easily moved in a direction of extension of the expansion body 21, and thus, storage capability can be improved.

The hand operation unit 23 has a housing 40 to be held by an operator, an operation dial 41 that can be rotationally operated by the operator, and a conversion mechanism 42 operated in conjunction with the rotation of the operation dial 41. The pulling shaft 26 is held by the conversion mechanism 42 inside the hand operation unit 23. The conversion mechanism 42 can move the held pulling shaft 26 forward and rearward in the axial direction in conjunction with the rotation of the operation dial 41. For example, a rack and pinion mechanism can be used as the conversion mechanism 42.

It is preferable that the shaft portion 20 is formed of a material having a certain degree of flexibility. Examples of such a material include polyolefin such as polyethylene, polypropylene, polybutene, ethylene-propylene copolymer, ethylene-vinyl acetate copolymer, ionomer, or a mixture of two or more of them, soft polyvinyl chloride resin, polyamide, polyamide elastomer, polyester, polyester elastomer, polyurethane, fluorine resin such as polytetrafluoroethylene, polyimide, PEEK, silicone rubber, and latex rubber.

The pulling shaft 26 can be formed of, for example, an elongated wire material including a super elasticity alloy such as a nickel-titanium alloy and a copper-zinc alloy, a metal material such as stainless steel, a resin material having comparatively high rigidity, or the like.

The distal end member 35 can be formed of, for example, a super elasticity alloy such as a nickel-titanium alloy or a copper-zinc alloy, a metal material such as stainless steel, a polymer material such as polyolefin, polyvinyl chloride, polyamide, polyamide elastomer, polyurethane, polyurethane elastomer, polyimide, or fluorine resin, or a mixture of the above polymer materials. Alternatively, the distal end member 35 can be formed of a multilayer tube containing two or more kinds of polymer materials.

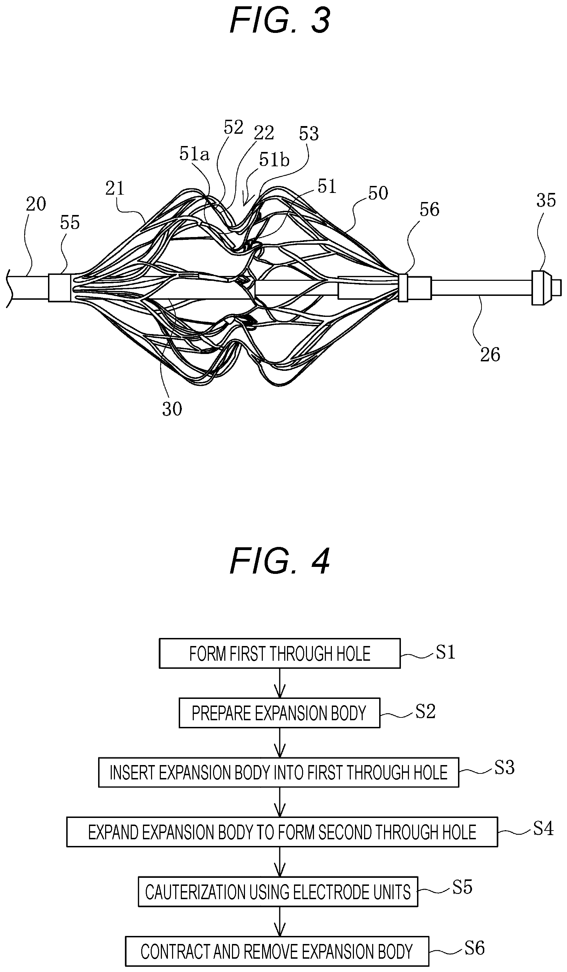

As illustrated in Figs. 2 and 3, the expansion body 21 includes a plurality of wire materials 50 in a circumferential direction. The wire materials 50 form a mesh-shaped structure by branching and joining along a length direction. As a result, the expansion body 21 can expand and contract in a radial direction. Proximal parts of the wire materials 50 extend to the distal side from a proximal convergence portion 55 of the expansion body 21. Distal parts of the wire materials 50 extend to the proximal side from a distal convergence portion 56 of the expansion body 21. In a state where the expansion body 21 is expanded, the wire materials 50 are inclined to expand in the radial direction from both ends toward central parts in the axial direction. Further, the wire materials 50 include a recess 51 in the central parts in the axial direction, the recess 51 being recessed radially inward of the expansion body 21. An innermost part of the recess 51 in the radial direction is a bottom 51a. The recess 51 defines a reception space 51b that can receive a biological tissue when the expansion body 21 is expanded.

The recess 51 includes a proximal-side upright portion 52 extending radially outward from the proximal end of the bottom 51a and a distal-side upright portion 53 extending radially outward from the distal end of the bottom 51a. When the pulling shaft 26 slides in the proximal direction with respect to the shaft portion 20 to apply a compressive force to the expansion body 21, the distal-side upright portion 53 and the proximal-side upright portion 52 are brought close to each other, and both portions come in close contact with the biological tissue received in the reception space 51b. The electrode unit 22 is disposed along the recess 51 at the proximal-side upright portion 52 so as to face the reception space 51b. In other words, the electrode unit 22 is disposed along the expansion body 21 in an intermediate part of the expansion body 21 in a central axis direction. In the present embodiment, ten electrode units 22 are disposed in the circumferential direction. Note that the electrode unit 22 may be disposed on the distal-side upright portion 53.

The wire materials 50 constituting the expansion body 21 can be formed by, for example, cutting a single metal cylindrical member with laser. The wire materials 50 can be formed of a metal material. Examples of the metal material that may be used include a titanium-based (Ti-Ni, Ti-Pd, Ti-Nb-Sn, etc.) alloy, a copper-based alloy, stainless steel, β-titanium steel, and a Co-Cr alloy. Note that it is more preferable to use an alloy having spring property such as a nickel titanium alloy. However, the wire materials 50 are not limited to be formed of the above materials, and may be formed of other materials.

The electrode units 22 are connected to an energy supply device (not illustrated) which is an external device. A high-frequency voltage is applied from the energy supply device to an electrode pair including two electrode units 22, and energy is applied between them. In other words, the electrode unit 22 is configured as a bipolar electrode. The electrode unit 22 may be a monopolar electrode. In this case, electric current is supplied between the electrode unit 22 and an external electrode.

As illustrated in Fig. 4, when the procedure of forming the communication hole Hh using the medical device 10 is performed, the first through hole Hh1 is formed in advance at the position of the fossa ovalis of the atrial septum HA (S1). Next, the medical device 10 having the expansion body 21 is prepared (S2), and the expansion body 21 in the contracted state is inserted into the first through hole Hh1 (S3). In the medical device 10, the expansion body 21 is expanded to widen the first through hole Hh1, by which the second through hole Hh2 that is substantially circular is formed (S4). Then, the edge of the second through hole Hh2 is cauterized by the electrode units 22 (S5), and the expansion body 21 is contracted and removed (S6). Thus, the communication hole Hh of which size, that is, patency, is maintained.

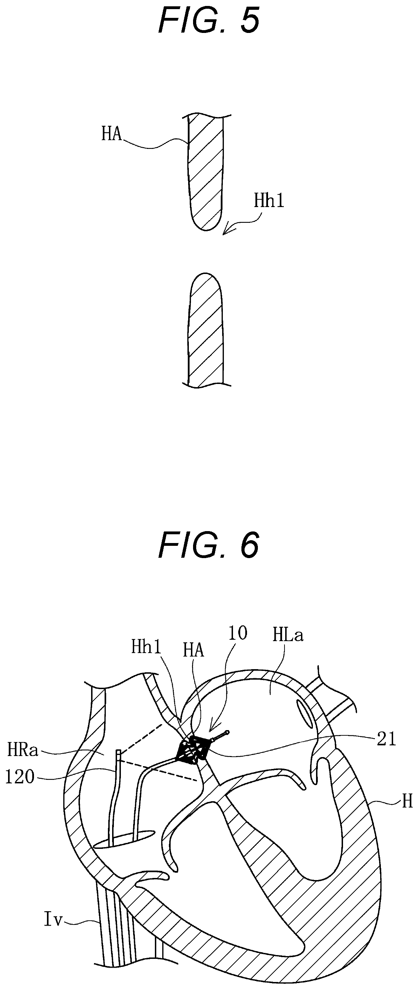

As illustrated in Fig. 5, in S1, the first through hole Hh1 is formed in the atrial septum HA. As illustrated in Fig. 6, in S3, the medical device 10 is delivered from an inferior vena cava Iv to the vicinity of the atrial septum HA via a right atrium HRa, and the expansion body 21 is placed at the position of the first through hole Hh1 that has been formed in advance. The medical device 10 is inserted such that the distal part thereof penetrates the atrial septum HA and reaches the left atrium HLa.

During the insertion of the medical device 10, the expansion body 21 is stored and contracted in the storage sheath 25, and after the storage sheath 25 penetrates the atrial septum HA, the storage sheath 25 is moved to the proximal side, by which the expansion body 21 can be exposed. When being exposed, the expansion body 21 radially expands, and the recess 51 is positioned at the first through hole Hh1 in the atrial septum HA and receives the biological tissue surrounding the first through hole Hh1 in the reception space 51b. The first through hole Hh1 is formed, for example, by opening a hole in the atrial septum HA using a puncture device and expanding the hole using a balloon catheter. The first through hole Hh1 thus formed has a substantially elliptical shape due to the influence of the orientation of the tissue of the atrial septum. For example, when the communication hole Hh having a diameter of 8 mm is formed, the major axis of the substantially elliptical first through hole Hh1, that is, the maximum distance between any two points on the outer edge of the first through hole Hh1, is about 8 mm. The first through hole Hh1 may be a hole formed in the atrial septum HA using a puncture device. In this case, the diameter of the first through hole Hh1 is within a range of about 1 to 2 mm.

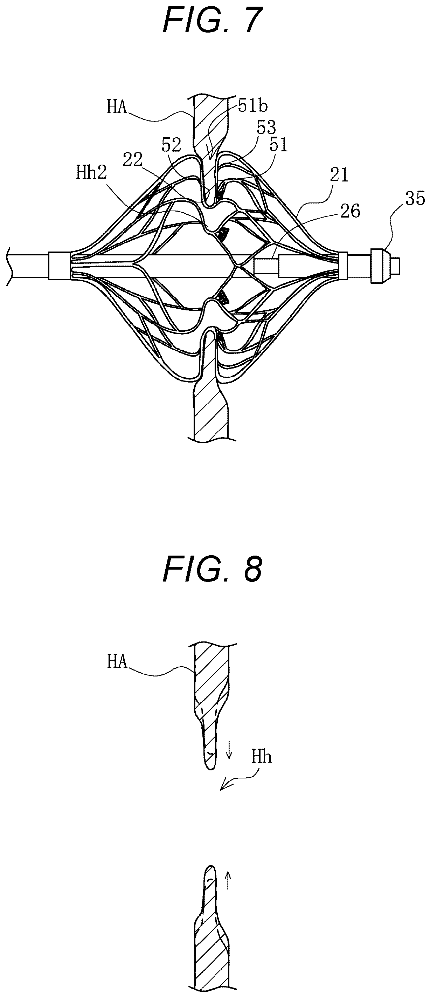

In S4, the pulling shaft 26 is moved to the proximal side in a state in which the reception space 51b receives the biological tissue, by which the expansion body 21 is pulled in a compression direction by the distal end member 35 to be compressed in the axial direction, the atrial septum HA is gripped by the proximal-side upright portion 52 and the distal-side upright portion 53 which constitute the recess 51, and the electrode units 22 are pressed against the biological tissue, as illustrated in Fig. 7. At this time, as the radial position of the recess 51 moves outward, the first through hole Hh1 is expanded in the radial direction, and the second through hole Hh2 is formed. The diameter of the second through hole Hh2 is larger than the diameter (major diameter) of the first through hole Hh1. For example, when the communication hole Hh having a diameter of approximately 8 mm is formed, the diameter of the second through hole Hh2 is within a range of 9 to 12 mm. The fossa ovalis where the second through hole Hh2 is formed has a smaller wall thickness than other parts of the atrial septum HA. Therefore, the recess 51 of the expansion body 21 can hold the biological tissue surrounding the periphery of the second through hole Hh2 in such a manner that the electrode units 22 are pressed against the biological tissue.

In a state where the electrode units 22 are pressed against the biological tissue, high frequency energy is applied to the edge of the second through hole Hh2, that is, the biological tissue surrounding the second through hole Hh2, through the electrode units 22, whereby the edge of the second through hole Hh2 can be cauterized (heated and cauterized) by the high frequency energy. The high frequency energy is applied by applying a voltage between the pair of electrode units 22 adjacent to each other in the circumferential direction. This results in making it possible to inhibit blockage of the communication hole Hh due to natural healing and maintain a size thereof. When the expansion body 21 is contracted and removed after the cauterization (S6), the second through hole Hh2 is slightly contracted in the radial direction to form the communication hole Hh as illustrated in Fig. 8. The diameter of the communication hole Hh of which size is maintained is 8 mm. Note that the communication hole Hh may not have a perfect circular shape due to the influence of the biological tissue surrounding the communication hole Hh. In this case, the diameter of the communication hole Hh refers to the maximum distance between any two points on the outer edge of the communication hole.

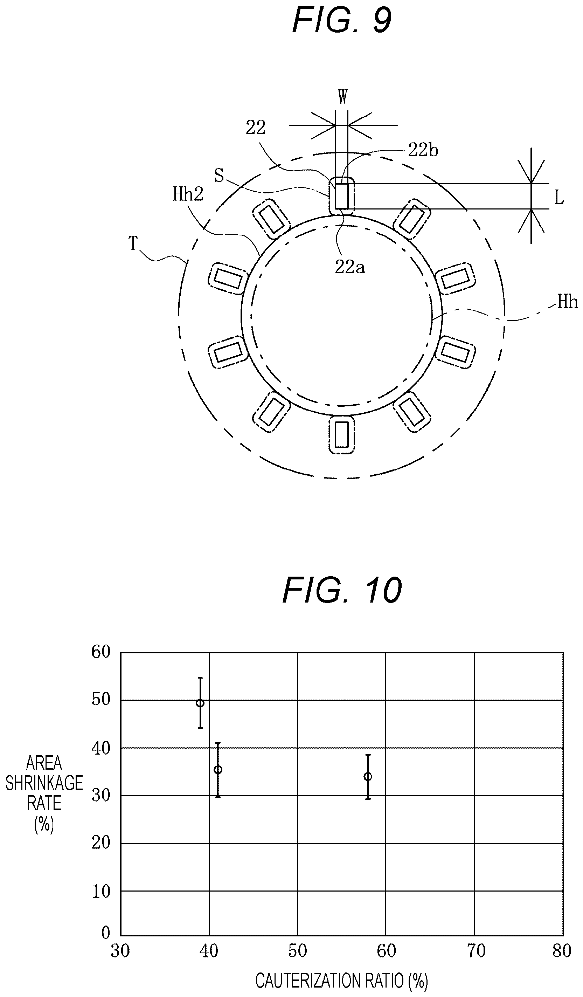

As illustrated in Fig. 9, in a state where the expansion body 21 grips the biological tissue, the ten electrode units 22 are arranged at equal intervals along the circumferential direction around the expanded second through hole Hh2. Each of the electrode units 22 cauterizes the biological tissue over a region of the biological tissue with which the electrode unit 22 is in contact and a region up to a distance of 0.5 mm from an edge of the electrode unit 22. In Fig. 9, a region S cauterized by each of the electrode units 22 is indicated by a dash-dot line. In addition, a region T of the fossa ovalis where the thickness of the biological tissue is small is indicated by a dash-dot-dot line.

The length of the electrode unit 22 along the direction of extension of the wire materials 50 is L (mm), and the width orthogonal to the length L (mm) of the electrode unit 22 is W (mm). The electrode unit 22 has a bottom-side end 22a on the bottom 51a side of the recess 51 of the expansion body 21 and an outer end 22b located on an opposite side to the bottom-side end 22a in the radial direction of the expansion body 21, and the length L (mm) of the electrode unit 22 is a length extending along the proximal-side upright portion 52 from the bottom-side end 22a to the outer end 22b (see Fig. 9). When the electrode unit 22 is disposed along the distal-side upright portion 53, the length L (mm) of the electrode unit 22 is a length extending along the distal-side upright portion 53 from the bottom-side end 22a to the outer end 22b. The average of the widths W (mm) of the plurality of electrode units 22 is defined as an average width Wa (mm). As described above, the edge of the region S cauterized by the electrode unit 22 is 0.5 mm away from the edge of the electrode unit 22, so that the length of the region S is L + 1 (mm), and the width of the region S is Wa + 1 (mm). When the edge of the electrode unit 22 coincides with the outer edge of the second through hole Hh2, the length of the region S is L + 0.5 (mm).

When the ratio of the total of the widths Wa (mm) of the regions S cauterized by the electrode units 22 to the circumferential length of the communication hole Hh is defined as a cauterization ratio P, P can be expressed by Expression (1) described below. The communication hole Hh is slightly smaller in diameter than the second through hole Hh2 expanded by the expansion body 21 as described above. The circumferential length of the communication hole Hh is based on the diameter of the communication hole Hh slightly contracted from the second through hole Hh2.

[Mathematical Expression 6]

$$P = \frac{(Wa + 1) \times N}{8\pi} \quad (1)$$

An experiment was conducted by changing the cauterization ratio P to compare the area shrinkage rate of the communication hole Hh after a certain period of time has elapsed from the cauterization. As illustrated in Fig. 10, it was found that when the cauterization ratio P was more than 40%, the area shrinkage rate did not change much, whereas when the cauterization ratio P was less than 40%, the area shrinkage rate increased. That is, the cauterization ratio P is desirably 40% or more. From this result, when the number N of the electrode units 22 and the average width Wa (mm) of the electrode units 22 satisfy Expression (2) described below, it is possible to reduce the possibility of the occurrence of recoil in which the formed shunt contracts in the short term.

[Mathematical Expression 7]

$$\frac{(Wa + 1) \times N}{8\pi} \geq 0.40 \quad (2)$$

In addition, the number N of the electrode units 22 and the average width Wa (mm) of the electrode units 22 more preferably satisfy Expression (3) described below, by which the possibility of the occurrence of recoil can be further reduced.

[Mathematical Expression 8]

$$\frac{(Wa + 1) \times N}{8\pi} \geq 0.60 \quad (3)$$

The length L (mm) of the electrode unit 22 is desirably set to cauterize the region T of the fossa ovalis. The reason is as follows. In the atrial septum HA, the thickness of the biological tissue sharply increases on the outside of the fossa ovalis, and thus when the outside of the fossa ovalis is cauterized, the volume to be cauterized increases, and a possibility of the occurrence of remodeling in which the shunt is blocked in the long term increases. The diameter of the fossa ovalis is within a range of 12 to 14 mm, and the diameter of the communication hole Hh is 8 mm as described above. In view of this, by setting the length L (mm) of the electrode unit 22 to 2.0 mm or less, the region S to be cauterized by the electrode units 22 can be kept approximately within the region T of the fossa ovalis. As a result, it is possible to reduce the possibility of the occurrence of remodeling.

In addition, by setting the length L (mm) of the electrode unit 22 to 0.7 mm or more, more preferably 1.0 mm or more, the area of the region S to be cauterized can be sufficiently ensured. Therefore, the length L (mm) of the electrode unit 22 satisfies Expression (4) described below, whereby the possibility of occurrence of remodeling can be decreased while sufficiently ensuring the area of the region S.

[Mathematical Expression 9]

$$0.7 \leq L \leq 2.0 \quad (4)$$

In addition, the electrode unit 22 is disposed so as to be in contact with the radially outer surface of the bottom 51a of the recess 51, and the length L (mm) of the electrode unit 22 is set to 1.5 mm or less, whereby the region S to be cauterized can be reliably kept within the region T of the fossa ovalis. In this case, the length L (mm) of the electrode unit is set to 0.7 mm or more, more preferably 1.0 mm or more, by which the area of the region S to be cauterized can be sufficiently ensured, and the region S to be cauterized can be reliably kept within the region T of the fossa ovalis.

In the present embodiment, the number N of the electrode units 22 is ten, the length L (mm) of each of the electrode units 22 is 1.0 mm, and the average width Wa (mm) of the electrode units 22 is 0.5 mm. In this case, the cauterization ratio P is 0.60, which satisfies the condition of Mathematical Expression 6. Further, the length L (mm) of each of the electrode units 22 satisfies the condition of Mathematical Expression 7. For this reason, the medical device 10 can reduce the possibility of the occurrence of recoil and remodeling that occur after cauterization.

When the communication hole Hh having a diameter of about 8 mm immediately after the cauterization is formed using the expansion body 21 in which the expansion force of the bottom 51a of the recess 51 in the expansion body 21 is within a range of 1.0 to 3.5 N in a state where the diameter of the bottom 51a of the recess 51 in the expansion body 21 is within a range of 10 to 12 mm, the distance from the central axis of the expansion body 21 to the outer end of each of the plurality of electrode units 22 in the radial direction of the expansion body 21 that is maximally expanded is within a range of 9 to 11 mm. Thus, the region S to be cauterized by the electrode units 22 can be kept approximately within the region T of the fossa ovalis by placing the recess 51 of the expansion body 21 in the communication hole Hh formed in the atrial septum HA using, for example, a balloon having a diameter of 14 mm.

When the medical device 10 is used, hemodynamics is checked by a hemodynamics checking device 120 delivered to the right atrium HRa via the inferior vena cava Iv. As the hemodynamics checking device 120, a known echo catheter can be used, for example. The operator can display an echo image obtained by the hemodynamics checking device 120 on a display device, such as a display, and can check the volume of blood passing through the communication hole Hh on the basis of a displayed result.

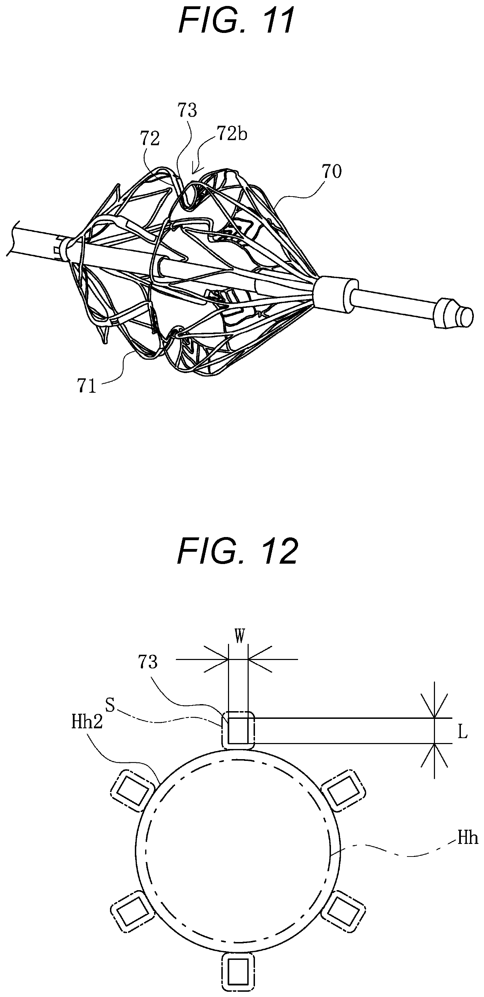

A modification of the expansion body in which the number N of electrode units is different will be described. As illustrated in Fig. 11, an expansion body 70 according to a first modification includes a plurality of wire materials 71, and has a recess 72 that is recessed radially inward at the time of expansion of the expansion body 70 and that defines a reception space 72b capable of receiving a biological tissue. The expansion body 70 has six electrode units 73 along the circumferential direction.

As illustrated in Fig. 12, the length L (mm) of each of the electrode units 73 along a direction of extension of the wire materials 71 is 1.0 mm, and the average width Wa (mm) orthogonal to the length L of the electrode unit 73 is 0.75 mm. The number N of the electrode units 73 is six, and thus, the cauterization ratio P in Expression (1) is 0.42 which satisfies the relationship represented by Expression (2). The length L of the electrode unit 73 satisfies Expression (4). Therefore, a medical device 10 having the six electrode units 73 in the expansion body 70 can reduce the possibility of occurrence of recoil and remodeling that occur after cauterization.

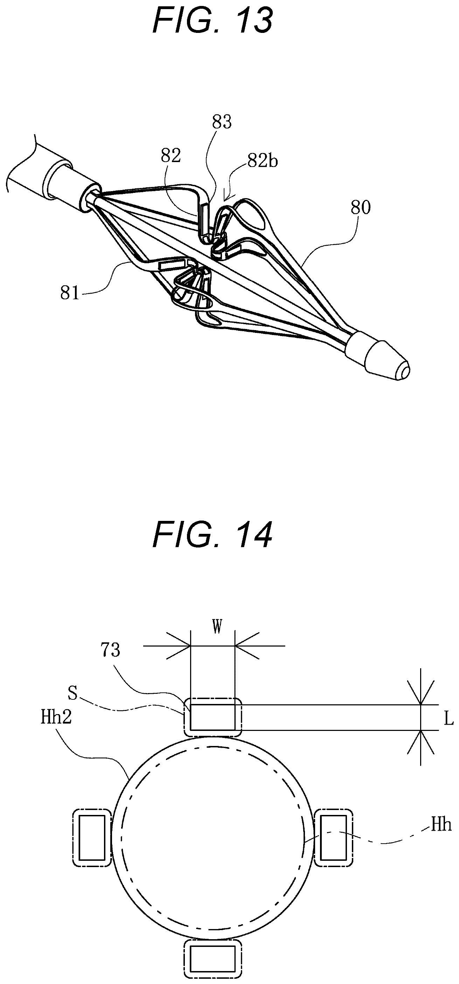

As illustrated in Fig. 13, an expansion body 80 according to a second modification includes a plurality of wire materials 81, and has a recess 82 that is recessed radially inward at the time of expansion of the expansion body 80 and that defines a reception space 82b capable of receiving a biological tissue. The expansion body 80 has four electrode units 83 along the circumferential direction.

As illustrated in Fig. 14, the length L (mm) of each of the electrode units 83 along a direction of extension of the wire materials 81 is 1.0 mm, and the average width Wa (mm) orthogonal to the length L of the electrode unit 83 is 1.75 mm. The number N of the electrode units 83 is four, and thus, the cauterization ratio P in Expression (1) is 0.44 which satisfies the relationship represented by Expression (2). The length L of the electrode unit 83 satisfies Expression (4). Therefore, a medical device 10 having the four electrode units 83 in the expansion body 80 can reduce the possibility of occurrence of recoil and remodeling that occur after cauterization.

As described above, (1) a medical device 10 according to the present embodiment is for forming a communication hole Hh in a biological tissue, the medical device 10 including: an expansion body 21 that is expandable and contractible in a radial direction; and a plurality of electrode units 22 disposed on the expansion body 21, wherein the expansion body 21 has a recess 51 that is recessed radially inward upon expansion of the expansion body 21 and that defines a reception space capable of receiving the biological tissue, the recess 51 includes a bottom 51a that is an innermost part in the radial direction of the expansion body 21, a proximal-side upright portion 52 extending radially outward from a proximal end of the bottom 51a, and a distal-side upright portion 53 extending radially outward from a distal end of the bottom 51a, the plurality of electrode units 22 extends along the proximal-side upright portion 52 or the distal-side upright portion 53 and is spaced apart from each other in a circumferential direction of the expansion body 21, each of the plurality of electrode units 22 includes a bottom-side end 22a located on a side of the bottom 51a of the recess 51 and an outer end 22b located on a side opposite to the bottom-side end 22a in the radial direction of the expansion body 21, and has a length L extending along the distal-side upright portion 52 or the proximal-side upright portion 53 from the bottom-side end 22a to the outer end 22b, and a width W (mm) orthogonal to the length L (mm), and a number N of the electrode units 22, the length L (mm) of each of the plurality of electrode units 22, and an average width Wa (mm) that is an average of the widths W (mm) of the plurality of electrode units 22 satisfy (Mathematical Expression 10) and (Mathematical Expression 11). In the medical device 10 configured as described above, the electrode units 22 can cauterize an appropriate region around the communication hole Hh, whereby it is possible to reduce the possibility of the occurrence of recoil in which the formed shunt contracts in the short term or remodeling in which the shunt is blocked in the long term due to strong healing reaction of the living body.

[Mathematical Expression 10]

$$L \leq 2.0$$

[Mathematical Expression 11]

$$\frac{(Wa + 1) \times N}{8\pi} \geq 0.40$$

(2) In the medical device 10 according to (1), the number N of the electrode units 22 and the average width W (mm) of the plurality of electrode units 22 may satisfy (Mathematical Expression 12). With this configuration, the medical device 10 cauterizes a wider region around the communication hole Hh, whereby it is possible to more reliably prevent the formed shunt from contracting in the short term.

[Mathematical Expression 12]

$$\frac{(Wa + 1) \times N}{8\pi} \geq 0.60$$

(3) In the medical device 10 according to (1) or (2), the number N of the electrode units 22 may be ten or more. With this configuration, the medical device 10 can satisfy the conditions represented by the above expressions even if the width of each electrode unit 22 is reduced, so that the flexibility of the expansion body 21 can be increased.

(4) In the medical device 10 according to any one of (1) to (3), the recess 51 may be deformed so as to crush and hold the biological tissue in a state in which the biological tissue is received in the reception space 51b. With this configuration, the medical device 10 can cauterize the biological tissue in an appropriate region when the biological tissue is held by the recess 51 so as to be crushed and is cauterized by the electrode units 22.

(5) In the medical device 10 according to any one of (1) to (4), the length L (mm) of each of the plurality of electrode units 22 may satisfy (Mathematical Expression 13). With this configuration, the medical device 10 can reduce the possibility of occurrence of remodeling while sufficiently ensuring the area to be cauterized by the electrode units 22.

[Mathematical Expression 13]

$$0.7 \leq L \leq 2.0$$

(6) In the medical device 10 according to any one of (1) to (5), each of the plurality of electrode units 22 may be disposed such that the bottom-side end is in contact with a radially outer surface of the bottom of the recess 51, and the length L (mm) of each of the plurality of electrode units 22 may satisfy (Mathematical Expression 14). With this configuration, the region to be cauterized by the electrode units 22 can be reliably kept within the region of the fossa ovalis.

[Mathematical Expression 14]

$$L \leq 1.5$$

(7) In the medical device 10 according to any one of (1) to (6), the plurality of electrode units 22 may be disposed at substantially equal intervals along the circumferential direction of the expansion body 21. With this configuration, the cauterization can be performed at equal intervals along the circumferential direction by the electrode units 22, whereby recoil that is short-term contraction can be further reduced.

(8) In the medical device 10 according to any one of (1) to (7), in a state in which the expansion body 21 is maximally expanded, the distance from a central axis of the expansion body 21 in the radial direction of the expansion body 21 to the outer end of each of the plurality of electrode units 22 may be within a range of 9 mm to 11 mm. With this configuration, it is possible to form a communication hole having a diameter of about 8 mm immediately after cauterization while keeping the region to be cauterized by the electrode units 22 approximately within the region of the fossa ovalis.

The method for forming a communication hole according to the present embodiment is (9) a method for forming a communication hole that allows communication between a right atrium and a left atrium in an atrial septum using an expansion body 21 that is expandable and contractible in a radial direction, the method including: preparing the expansion body 21 including a recess 51 that is recessed radially inward upon expansion of the expansion body 21 and that defines a reception space capable of receiving a biological tissue; inserting the expansion body 21 into a first through hole Hh1 formed in the atrial septum HA to place a tissue surrounding the first through hole Hh1 in the reception space defined by the recess 51; expanding the recess 51 of the expansion body 21 in the radial direction to expand the first through hole Hh1 to a second through hole Hh2 larger than the communication hole Hh; cauterizing the tissue surrounding the second through hole Hh2 with a plurality of electrode units 21 disposed in the recess 51 of the expansion body 21 in such a manner that a cauterization region of the tissue surrounding the communication hole Hh has a length of 3.0 mm or less extending in the radial direction from an edge of the communication hole Hh and a ratio of 40% or more to a circumferential length of the edge of the communication hole Hh in a circumferential direction; and forming the communication hole Hh by contracting and removing the expansion body 21 from the second communication hole Hh2 after the cauterization of the tissue surrounding the second through hole Hh2. With the method for forming the communication hole Hh configured as described above, an appropriate region suitable for the diameter of the communication hole Hh slightly contracted from the second through hole Hh2 after the removal of the expansion body 21 can be cauterized by the electrode units 22. Thus, it is possible to reduce the possibility of the occurrence of recoil in which the formed shunt contracts in the short term or remodeling in which the shunt is blocked in the long term due to strong healing reaction of the living body.

Note that the present invention is not limited to the embodiment described above, and those skilled in the art can make various modifications within the technical idea of the present invention.

Note that the present application is based on Japanese Patent Application No. 2023-155455 filed on September 21, 2023, the entire disclosed content of which is incorporated herein by reference.

Reference Signs List

10 Medical device

| 11 | Guide wire |
|---|---|
| 20 | Shaft portion |
| 21 | Expansion body |
| 22 | Electrode unit |
| 23 | Hand operation unit |
| 25 | Storage sheath |
| 26 | Pulling shaft |
| 27 | Curved portion |
| 30 | Distal shaft portion |
| 35 | Distal end member |
| 40 | Housing |
| 50 | Wire material |
| 51 | Recess |
| 51b | Reception space |
| 52 | Proximal-side upright portion |
| 53 | Distal-side upright portion |
| 55 | Proximal convergence portion |
| 56 | Distal convergence portion |
| H | Heart |
| Hh | Communication hole |

**Claims**

1. A medical device for forming a communication hole in a biological tissue, the medical device comprising:

   an expansion body that is expandable and contractible in a radial direction; and
   a plurality of electrode units disposed on the expansion body, wherein
   the expansion body has a recess that is recessed radially inward upon expansion of the expansion body and that defines a reception space capable of receiving the biological tissue,
   the recess includes a bottom that is an innermost part in the radial direction of the expansion body, a proximal-side upright portion extending radially outward from a proximal end of the bottom, and a distal-side upright portion extending radially outward from a distal end of the bottom,
   the plurality of electrode units extends along the proximal-side upright portion or the distal-side upright portion and is spaced apart from each other in a circumferential direction of the expansion body,
   each of the plurality of electrode units includes a bottom-side end located on a side of the bottom of the recess and an outer end located on a side opposite to the bottom-side end in the radial direction of the expansion body, and has a length L extending along the distal-side upright portion or the proximal-side upright portion from the bottom-side end to the outer end, and a width W (mm) orthogonal to the length L (mm), and
   a number N of the electrode units, the length L (mm) of each of the plurality of electrode units, and an average width Wa (mm) that is an average of the widths W (mm) of the plurality of electrode units satisfy (Mathematical Expression 1) and (Mathematical Expression 2).

[Mathematical Expression 1]

$$L \leq 2.0$$

[Mathematical Expression 2]

$$\frac{(Wa + 1) \times N}{8\pi} \geq 0.40$$

2. The medical device according to claim 1, wherein the number N of the electrode units and the average width Wa (mm) of the plurality of electrode units satisfy (Mathematical Expression 3).

[Mathematical Expression 3]

$$\frac{(Wa + 1) \times N}{8\pi} \geq 0.60$$

3. The medical device according to claim 2, wherein the number N of the electrode units is ten or more.

4. The medical device according to claim 3, wherein the recess is deformed so as to crush and hold the biological tissue in a state in which the biological tissue is received in the reception space.

5. The medical device according to any one of claims 1 to 4, wherein the length L (mm) of each of the plurality of electrode units satisfies (Mathematical Expression 4).

[Mathematical Expression 4]

$$0.7 \leq L \leq 2.0$$

6. The medical device according to any one of claims 1 to 4, wherein

   each of the plurality of electrode units is disposed in such a manner that the bottom-side end is in contact with a radially outer surface of the bottom of the recess, and
   the length L (mm) of each of the plurality of electrode units satisfies (Mathematical Expression 5).

[Mathematical Expression 5]

$$L \leq 1.5$$

7. The medical device according to any one of claims 1 to 4, wherein the plurality of electrode units is disposed at substantially equal intervals along a circumferential direction of the expansion body.

8. The medical device according to any one of claims 1 to 4, wherein, in a state in which the expansion body is maximally expanded, a distance from a central axis of the expansion body in the radial direction of the expansion body to the outer end of each of the plurality of electrode units is within a range of 9 mm to 11 mm.

9. A method for forming a communication hole that allows communication between a right atrium and a left atrium in an atrial septum using an expansion body that is expandable and contractible in a radial direction, the method comprising:

   preparing the expansion body including a recess that is recessed radially inward upon expansion of the expansion body and that defines a reception space capable of receiving a biological tissue;
   inserting the expansion body into a first through hole formed in the atrial septum to place a tissue surrounding the first through hole in the reception space defined by the recess;
   expanding the recess of the expansion body in the radial direction to expand the first through hole to a second through hole larger than the communication hole;
   cauterizing the tissue surrounding the second through hole with a plurality of electrode units disposed in the recess of the expansion body in such a manner that a cauterization region of the tissue surrounding the communication hole has a length of 3.0 mm or less extending in the radial direction from an edge of the communication hole and a ratio of 40% or more to a circumferential length of the edge of the communication hole in a circumferential direction; and
   forming the communication hole by contracting and removing the expansion body from the second communication hole after the cauterization of the tissue surrounding the second through hole.

# FIG. 1

# FIG. 2

## FIG. 3

## FIG. 4

| | |
|---|---|
| FORM FIRST THROUGH HOLE | S1 |
| PREPARE EXPANSION BODY | S2 |
| INSERT EXPANSION BODY INTO FIRST THROUGH HOLE | S3 |
| EXPAND EXPANSION BODY TO FORM SECOND THROUGH HOLE | S4 |
| CAUTERIZATION USING ELECTRODE UNITS | S5 |
| CONTRACT AND REMOVE EXPANSION BODY | S6 |

*FIG. 5*

*FIG. 6*

## FIG. 7

## FIG. 8

# FIG. 9

# FIG. 10

# FIG. 11

# FIG. 12

# FIG. 13

83

82        82b

80

81

# FIG. 14

W

73

S

Hh2

L

Hh

## INTERNATIONAL SEARCH REPORT

| International application No. |
|---|
| **PCT/JP2024/032326** |

| A. | CLASSIFICATION OF SUBJECT MATTER |
|---|---|

*A61B 18/12*(2006.01)i; *A61B 18/14*(2006.01)i
FI: A61B18/12; A61B18/14

According to International Patent Classification (IPC) or to both national classification and IPC

| B. | FIELDS SEARCHED |
|---|---|

Minimum documentation searched (classification system followed by classification symbols)

A61B18/12; A61B18/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2022-143033 A (TERUMO KABUSHIKI KAISHA) 03 October 2022 (2022-10-03) paragraphs [0036]-[0093], fig. 1-4, 6-19 | 1-9 |
| A | JP 2007-526087 A (NMT MEDICAL, INC.) 13 September 2007 (2007-09-13) entire text, all drawings | 1-9 |
| A | WO 2019/189079 A1 (TERUMO KABUSHIKI KAISHA) 03 October 2019 (2019-10-03) entire text, all drawings | 1-9 |
| A | JP 2008-521505 A (ABLATION FRONTIERS, INC.) 26 June 2008 (2008-06-26) entire text, all drawings | 1-9 |
| A | JP 2016-524949 A (BOSTON SCIENTIFIC SCIMED, INC.) 22 August 2016 (2016-08-22) entire text, all drawings | 1-9 |
| A | JP 2023-090689 A (BIOSENSE WEBSTER (ISRAEL), LTD.) 29 June 2023 (2023-06-29) entire text, all drawings | 1-9 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 October 2024** | **15 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**EP 4 781 931 A1**

## INTERNATIONAL SEARCH REPORT

International application No.

**PCT/JP2024/032326**

### C.    DOCUMENTS CONSIDERED TO BE RELEVANT

| Category* | Citation of document, with indication, where appropriate, of the relevant passages | Relevant to claim No. |
|---|---|---|
| A | JP 2021-523755 A (ST. JUDE MEDICAL, CARDIOLOGY DIVISION, INC.) 09 September 2021 (2021-09-09)<br>entire text, all drawings | 1-9 |
| A | US 2013/0331920 A1 (OSYPKA, Peter) 12 December 2013 (2013-12-12)<br>entire text, all drawings | 1-9 |
| A | JP 2023-125512 A (JAPAN LIFELINE CO., LTD.) 07 September 2023 (2023-09-07)<br>entire text, all drawings | 1-9 |
| A | JP 2016-10729 A (COVIDIEN LP) 21 January 2016 (2016-01-21)<br>entire text, all drawings | 1-9 |
| A | US 2020/0170662 A1 (INTERSHUNT TECHNOLOGIES, INC.) 04 June 2020 (2020-06-04)<br>entire text, all drawings | 1-9 |
| A | JP 2022-042115 A (TERUMO KABUSHIKI KAISHA) 14 March 2022 (2022-03-14)<br>entire text, all drawings | 1-9 |
| A | WO 2022/071169 A1 (TERUMO KABUSHIKI KAISHA) 07 April 2022 (2022-04-07)<br>entire text, all drawings | 1-9 |

Form PCT/ISA/210 (second sheet) (July 2022)

21

## INTERNATIONAL SEARCH REPORT
### Information on patent family members

International application No.

**PCT/JP2024/032326**

| Patent document cited in search report | | | Publication date (day/month/year) | Patent family member(s) | | Publication date (day/month/year) |
|---|---|---|---|---|---|---|
| JP | 2022-143033 | A | 03 October 2022 | (Family: none) | | |
| JP | 2007-526087 | A | 13 September 2007 | US 2005/0267523 entire text, all drawings WO 2005/092203 | A1 A1 | |
| WO | 2019/189079 | A1 | 03 October 2019 | US 2021/0007791 entire text, all drawings EP 3777741 | A1 A1 | |
| JP | 2008-521505 | A | 26 June 2008 | US 2006/0111701 entire text, all drawings WO 2006/058253 | A1 A1 | |
| JP | 2016-524949 | A | 22 August 2016 | US 2014/0378967 entire text, all drawings WO 2014/205399 CN 105473092 | A1 A1 A | |
| JP | 2023-090689 | A | 29 June 2023 | US 2023/0190365 entire text, all drawings EP 4197473 CN 116264986 | A1 A1 A | |
| JP | 2021-523755 | A | 09 September 2021 | US 2021/0153932 entire text, all drawings WO 2019/195439 CN 112040861 | A1 A1 A | |
| US | 2013/0331920 | A1 | 12 December 2013 | EP 2674189 entire text, all drawings | A1 | |
| JP | 2023-125512 | A | 07 September 2023 | US 2023/0270490 entire text, all drawings DE 102023100781 CN 116650101 | A1 A1 A | |
| JP | 2016-10729 | A | 21 January 2016 | US 2014/0243821 entire text, all drawings WO 2013/049601 CN 103826694 KR 10-2014-0053377 | A1 A2 A A | |
| US | 2020/0170662 | A1 | 04 June 2020 | WO 2015/192109 entire text, all drawings | A1 | |
| JP | 2022-042115 | A | 14 March 2022 | (Family: none) | | |
| WO | 2022/071169 | A1 | 07 April 2022 | US 2023/0277235 entire text, all drawings EP 4212200 | A1 A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020094094 A

- JP 2023155455 A